# EUROPEAN PATENT SPECIFICATION

(11) **EP 2 494 913 B1**
(45) Date of publication and mention of the grant of the patent: **12.12.2018**
(21) Application number: 10826068.8
(22) Date of filing: 25.10.2010
(51) Int. Cl.: A61B 1/31, A61M 29/00, A61B 17/00, A61B 1/06, A61B 17/02, A61B 1/32

(54) **ANORECTAL SURGICAL INSTRUMENT AND ANAL DILATOR**
ANOREKTALES OPERATIONSINSTRUMENT UND ANALER DILATATOR
INSTRUMENT CHIRURGICAL ANO-RECTAL ET DILATATEUR ANAL

(30) Priority: 26.10.2009 CN 200920220138 U; 26.10.2009 CN 200920220139 U; 21.10.2010 CN 201020570951 U
(43) Date of publication of application: 05.09.2012
(73) Proprietor: B. J. Zh. F. Panther Medical Equipment Co., Ltd., Beijing 100018 (CN)
(72) Inventor: LI, Xuejun, Beijing, 102200 (CN); LIU, Qing, Beijing, 102200 (CN)
(74) Representative: AWA Sweden AB
(86) International application number: PCT/CN2010/078058
(87) International publication number: WO 2011/050705

(56) References cited:
- EP-A1- 1 929 934
- EP-A1- 1 929 934
- EP-A2- 1 234 539
- WO-A1-2006/033122
- CN-A- 1 717 195
- CN-A- 101 147 690
- CN-A- 101 355 903
- CN-U- 201 519 170
- CN-Y- 2 476 253
- CN-Y- 2 476 253
- US-A1- 2003 130 559
- US-A1- 2003 163 026

## Description

### FIELD OF THE INVENTION

The present application relates to the technical field of medical instruments, in particular, to a surgical instrument used in surgical operations for treating anorectal diseases. The present application further relates to an anal dilator in the surgical instrument.

### BACKGROUND OF THE INVENTION

The hemorrhoid is one of the common diseases. As the saying goes, nine of ten men may suffer from hemorrhoids. The incidence of hemorrhoids is high, and is about 85% of all anorectal diseases. To explain causes of hemorrhoids, the theory of "anal canal vascular cushion", referred to as "anal cushion theory" for short, has been developed since 1960. In the theory, it is considered that the anal cushion is an annular tissue band having a width from 1.5cm to 2.0cm above the dentate line in the anal canal of the human body. The tissue band is thick and soft, is a highly specified vascular cushion, and is a normal tissue in the human body. However, the vascular cushion may become pathologic hypertrophy due to diarrhea, constipation, increased intra-abdominal pressure and so on, and then move down, thereby resulting in the hemorrhoid.

Therapeutic means for hemorrhoids has been changed greatly as the above concept is widely accepted. In the "TENTATIVE STANDARD FOR HEMORRHOID DIAGNOSIS" made by the Anorectal Science Group of the Surgery Society of the Chinese Medical Association in 2000, the hemorrhoid is classed into four classes, i.e., I to IV degree. At present, it is hold that the moderate hemorrhoid at III degree and the severe hemorrhoid at IV degree must be treated through surgical operation. The therapeutic means of "Procedure for Prolapse and Hemorrhoid" (PPH surgery), which is originated from 1990s, is most widely accepted. The surgical operation is to cut out the mucosa and submucosa located at the level from 2 to 4 cm above the dentate line, such that the anal cushion is moved upwardly and is hung, thereby significantly alleviating the prolapse symptom. Meanwhile, hemorrhoid body is atrophied after the surgical operation, since blood supply of arterial branches in the rectum to the hemorrhoid region is cut off by cutting out the tissues under the mucosal. The surgical operation is favorable all over the word due to some advantages, for example, painless after the surgical operation, simple in the surgical operation, and no anal incontinence.

At present, in the surgical operation for treating anorectal diseases, medical instruments used in the PPH operation include a hemorrhoid stapler, and fittings such as an anal dilator and a threading device, which must be used simultaneously as a complete set. Thus, the anal dilator is a necessary instrument in the surgical operation.

During the surgical operation, the visual field at the anus is not good, it is inconvenient, even unsafe, to use the existing anal dilator. It can be seen that, the visual field of the surgical operation is illuminated by the anal dilator which has a diameter of about 4 cm and a depth above 8 cm in all the procedures, thus the visual field is considerably unclear. The surgeon sometimes needs an additional headlight to illuminate the surgical visual field, but sometimes, the visual field is still unclear. Since the space is small and the visual field is unclear, it is hard to perform the purse-string suture, hard to check whether a cut tissue has entirely entered the staple cartridge of the hemorrhoid stapler, and is impossible to visually determine whether other tissues, for example, the vagina of the female or a muscle layer of the intestinal canal, has entered into the opening of the stapler. Thus, these determinations can be made depending on the surgeon's experience. Once the surgeon makes a mistaken determination, it will lead to severe complication, for example, massive hemorrhage, anal vaginal fistula, postoperative stenosis and so on.

Methods for improving high-precision operations and to avoid mistakes depending on the skillfulness or experience of the surgeon are mentioned for example in WO06033122 A1, however, the range of the visual field is still an issue. A method for improving the visibility for the surgeon is mentioned in EP1929934B1, but still provides for limited visibility. Further improvements to an anal hemorrhoid stapler which reduces the costs of the surgical operations are disclosed in CN2476253Y.

Thus, a major technical problem to be solved by the person skilled in the art presently is to improve the range of the visual field of the anus during the surgical operation, so as to enhance convenience and safety of the surgical operation.

### SUMMARY OF THE INVENTION

An object of the present application is to provide an anal dilator which is made of transparent materials and is provided with a luminescent light source. During a surgical operation, an operative field, especially a deep visual field, can be entirely illuminated for surgeons by the luminescent light source on the anal dilator, so as to greatly enhance convenience and safety of the surgical operation.

Another object of the present application is to provide an anorectal surgical instrument provided with the anal dilator.

The invention is defined in independent claim 1 and the dependent claims 2 - 7.

The anal dilator according to the present application is further improved on the basis of the prior art. In view of the problems of the existing anal dilator, for example, inconvenient operation and low safety and reliability caused by unclear visual field, the external light source assembly is provided at the enlarged edge of the anal dilator according to the present application. The luminescent light source of the external light source assembly can emit a visible light with sufficient intensity. Since the cylindrical body is made of transparent materials, the visible light can illuminate the entire operative field, in particular, the deep visual field, through the wall or the light channel of the cylindrical body, which seems as if the cylindrical body had the luminescence function, thereby ensuring the safety and success of the surgical operation. In addition, since the light source assembly is arranged outside the cylindrical body, it facilitates operating and controlling without negative influence on application effects of the anal dilator.

The disposable hemorrhoid stapler for the anorectal surgical instrument according to the present application has a transparent staple cartridge, so as to allow an operator to clearly observe the range that a tissue to be resected enters an opening of the hemorrhoid stapler and a status that the tissue is lifted, and to determine whether a tissue that should not enter has been avoided, so as to ensure the excision range is accurate and complete and to increase the success rate of the surgical operation.

### BRIEF DESCRIPTION OF THE DRAWINGS

Figure 1 is an outline view of a first example of an anal dilator;
Figure 2 is a structural view of the anal dilator shown in figure 1;
Figure 3 is an outline view of a second example of an anal dilator;
Figure 4 is a structural view of the anal dilator shown in figure 3;
Figure 5 is an outline view of an embodiment of the anal dilator according to the present application;
Figure 6 is a structural schematic view of an external light source assembly shown in figure 5;
Figure 7 is a sectional view of the cylindrical body shown in figure 5;
Figure 8 is a partial enlarged schematic view of part I in figure 7;
Figure 9 is a structural schematic view of the anal dilator shown in figure 5, with the external light source assembly being removed;
Figure 10 is an outline view of a disposable hemorrhoid stapler according to the present application;
Figure 11 is a partial enlarged schematic view of a staple cartridge sleeve in figure 10 ;
Figures 12 is an exploded schematic view of an internal structure of the stapler shown in figure 10; and
Figure 13 is a schematic view of the assembly of the disposable hemorrhoid stapler and the anal dilator according to the present application.

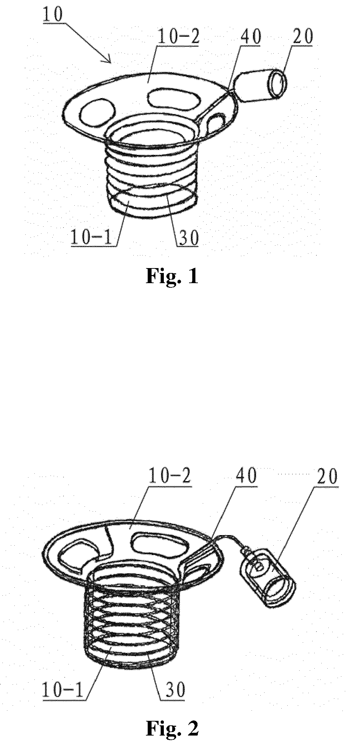

In figures 1 to 4:

| | | |
|---|---|---|
| 10. anal dilator | 10-1. cylindrical body | 10-2. enlarged edge |
| 20. light source assembly | 30. optical fiber | 40. light source interface |

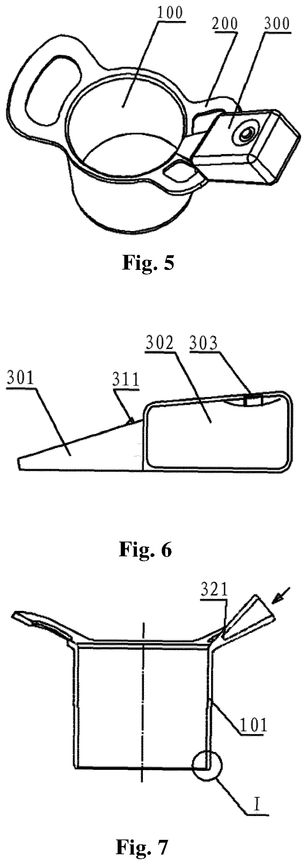

In figures 5 to 9:

| | | |
|---|---|---|
| 100. cylindrical body | 101. light channel | 121. light emitting opening |
| 200. enlarged edge | 201. wedge-shaped hole | 211. aperture |
| 300. external light source assembly | | 301. light-concentrating head |
| 302. battery | | 303. power switch |
| 311. slope convex block | | 321. light incident opening |

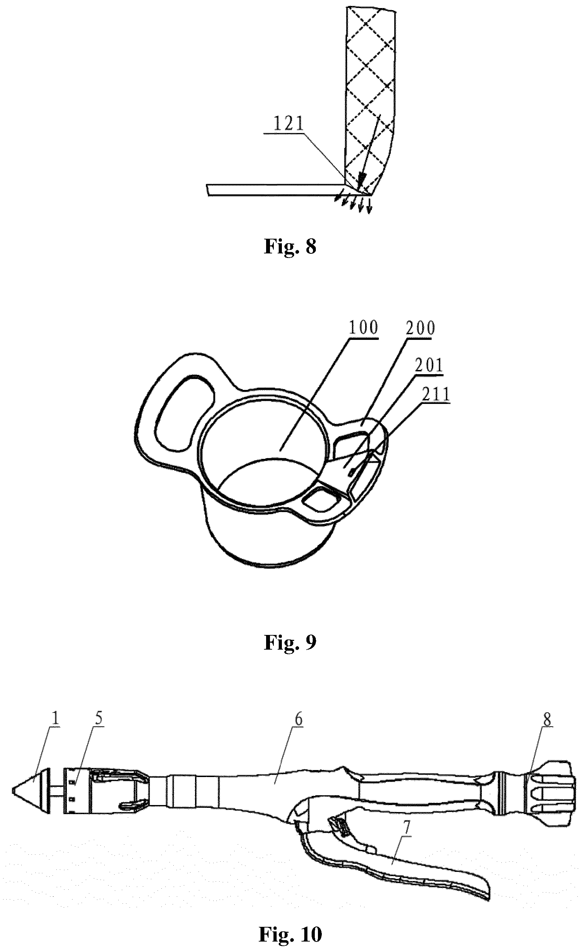

In figures 10 to 13:

| | | |
|---|---|---|
| 1. anvil | 2. annular knife gasket | 3. staple cartridge |
| 4. staple-pushing member | 5. staple cartridge sleeve | 5-1. operating hole |
| 5-2. thread-hooking hole | 6. gun body assembly | 7. movable handle |
| 8. adjusting nut | | |

### DETAILED DESCRIPTION

An aspect of the present application is to provide an anal dilator which is made of transparent materials and is provided with a luminescent light source. During a surgical operation, an operative field, especially a deep visual field, can be entirely illuminated for surgeons by the luminescent light source on the anal dilator, which greatly enhances convenience and safety of the surgical operation. Another aspect of the present application is to provide an anorectal surgical instrument provided with the anal dilator.

In order that persons skilled in the art could better understand technical solutions of the present application, hereinafter, the present application will be described further in conjunction with the accompanying drawings and specific embodiments.

Referring to figures 1 and 2, figure 1 is an outline view of a first example of an anal dilator, and figure 2 is a structural view of the anal dilator shown in figure 1.

In the first example, the anal dilator 10 includes a transparent cylindrical body 10-1, with the cylindrical body 10-1 being provided on its top portion with an elastic enlarged edge 10-2. An outer contour of the whole anal dilator 10 is of an inverted hat shape, with the cylindrical body 10-1 corresponding to a hat body and the trumpet-shaped enlarged edge 10-2 corresponding to a hat edge. The cylindrical body 10-1 is marked with legible scales to facilitate observation and surgical operation of the surgeon. The trumpet-shaped enlarged edge 10-2 is uniformly provided with several hollow-out portions. A light source assembly 20 is arranged outside the enlarged edge 10-2. An optical fiber 30 is wound circumferentially around an outer wall of the cylindrical body 10-1 and is connected to the light source assembly 20 via a light source interface 40 on the enlarged edge 10-2.

In particular, the optical fiber 30 employs a luminescent plastic fiber having a diameter from 0.25 mm to 0.5 mm. The light source assembly 20 mainly includes a housing, and a luminescent light source and a battery which are located in the housing. The luminescent light source employs a cold light source, for example, a high-brightness light emitting diode with a size of 2×2, 2×3, 2×4 and 2×5 mm, and a diameter ranged from 3 mm to 5 mm. The battery may employ a button cell having a working voltage from 1.5 V to 5 V.

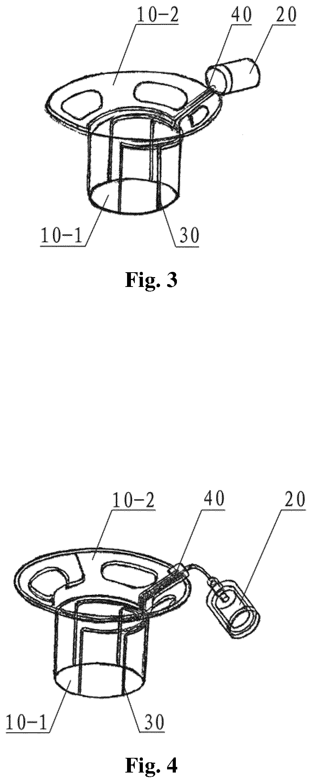

Referring to figures 3 and 4, figure 3 is an outline view of a second example of an anal dilator, and figure 4 is a structural view of the anal dilator shown in figure 3.

In the second example, the anal dilator 10 includes a transparent cylindrical body 10-1, with the cylindrical body 10-1 being provided on its top portion with an elastic enlarged edge 10-2. An outer contour of the whole anal dilator 10 is of an inverted hat shape, with the cylindrical body 10-1 corresponding to a hat body and the trumpet-shaped enlarged edge 10-2 corresponding to a hat edge. The cylindrical body 10-1 is marked with legible scales to facilitate observation and surgical operation of the surgeon. The trumpet-shaped enlarged edge 10-2 is made of medical rubber material and is uniformly provided with several hollow-out portions. A light source assembly 20 is arranged outside the enlarged edge 10-2. An optical fiber 30 is arranged, in a longitudinal direction of the cylindrical body 10-1, on the outer wall of the cylindrical body 10-1 and is connected to the light source assembly 20 via a light source interface 40 on the enlarged edge 10-2.

In particular, the optical fiber 30 employs a luminescent plastic fiber having a diameter from 0.25 mm to 0.5 mm. The light source assembly 20 mainly includes a housing, and a luminescent light source and a battery which are located in the housing. The luminescent light source employs a cold light source, for example, a high-brightness light emitting diode with a size of 2×2, 2×3, 2×4 and 2×5 mm, and a diameter ranged from 3 mm to 5 mm. The battery may employ a button cell having a working voltage from 1.5 V to 5 V.

Preferably, the optical fiber 30 employs a side-glowing optical fiber. More preferably, the optical fiber 30 may be formed into a single strand or a mesh shape. The mesh-shaped optical fiber is composed of side-glowing optical fibers having small diameters, which may form a flexible light band.

The above optical fiber may be located on an outer wall or an inner wall of the cylindrical body, or may be located between the outer wall and the inner wall of the cylindrical body. For example, the optical fiber may be wound circumferentially around the inner wall of the cylindrical body, or may be arranged, in the longitudinal direction of the cylindrical body, on the inner wall of the cylindrical body. In particular, fine grooves may be provided in the inner wall or the outer wall of the cylindrical body, or may be provided between the inner wall and the outer wall of the cylindrical body, such that the optical fiber can be embedded in the fine grooves. It is desirable to directly integrate the optical fiber between the inner wall and the outer wall of the cylindrical body by winding or arranging the optical fiber during manufacturing process of the cylindrical body, which would not adversely affect the operational performance of the cylindrical body, but has certain requirements in the processing of the cylindrical body.

Referring to figures 5 to 9, figure 5 is an outline view an embodiment of the anal dilator according to the present application, figure 6 is a structural schematic view of an external light source assembly shown in figure 5, figure 7 is a sectional view of the cylindrical body shown in figure 5, figure 8 is a partial enlarged schematic view of part I in figure 7, and figure 9 is a structural schematic view of the anal dilator shown in figure 5, with the external light source assembly being removed.

In the embodiment, the anal dilator according to the present application includes a transparent cylindrical body 100, with the cylindrical body 100 being provided on its top portion with an elastic enlarged edge 200. The enlarged edge 200 is provided with two lateral wings, one of which is provided with a wedge-shaped hole 201. The cylindrical body 100 and the enlarged edge 200 are both made of transparent materials, and are formed into a double-layer structure inside which a gap is formed. The gap inside the double-layer structure forms a light channel. A wedge-shaped light-concentrating head 301 is provided at a front end of a luminescent light source of an external light source assembly 300. A slope convex block 311 is provided on a top surface of the light-concentrating head 301 and is adjacent to a root portion of light-concentrating head 301, while a corresponding aperture 211 is provided in a top wall of the wedge-shaped hole 201. In use, the light-concentrating head 301 is inserted into the wedge-shaped hole 201 of the enlarged edge 200. The slope convex block 311 can be elastically embedded into the aperture 211 when the light-concentrating head 301 is fitted into the wedge-shaped hole 201 in position, such that the external light source assembly 300 can be fixedly connected to the enlarged edge 200.

The external light source assembly 300 mainly includes the light-concentrating head 301, a light emitting diode (not shown in the figures), a battery 302 and a power switch 303. Two electrodes of the light emitting diode are connected to a positive electrode and a negative electrode of the battery 302 via electric wires, respectively, and the light emitting diode protrudes out of a rectangular housing and into the light-concentrating head 301. The battery 302 employs a button cell having a working voltage from 1.5V to 5V. The battery, together with the power switch 303, is mounted in the rectangular housing, with an operating end of the power switch 303 protruding out of the housing.

A visible light from the light emitting diode passes through a light incident opening 321 of a front end of the light-concentrating head 301 into a light channel 101 of the cylindrical body 100, such that an inner portion of the transparent cylindrical body is filled with light rays. The light rays of the visible light form light paths within the cylindrical body, and illuminate the entire operative field after transmitting through and being continuously reflected (in the figures, the arrows indicate directions of the light rays, and the dotted lines indicate reflective paths of the light rays) by the inner wall of the cylindrical body. Particularly, a contracted light emitting opening 121 is provided at a bottom end of the cylindrical body 100, and the light rays transmit through the light emitting opening 121 to illuminate a deep visual field during the surgical operation.

Of course, the wall of the cylindrical body also may be a transparent solid body that can guide light and transmit light, and the operative field may be illuminated by the external light source assembly through the wall of the cylindrical body. The other structures are substantially the same with the embodiment mentioned above and will not be described repeatedly. This cylindrical body with the structure has a lower requirement of processing, which facilitates saving the manufacture cost and increasing the production efficiency.

Besides the anal dilator, the present application also provides an anorectal surgical instrument which includes a disposable hemorrhoid stapler and fittings such as the anal dilator and a thread-hooking device. With respect to a detailed structure of the anal dilator, reference may be made to the above detailed description, and with respect to other fittings such as the thread-hooking device, reference may be made to the prior art, which will not be described herein. Hereinafter, a detailed description will be made to an improved disposable hemorrhoid stapler.

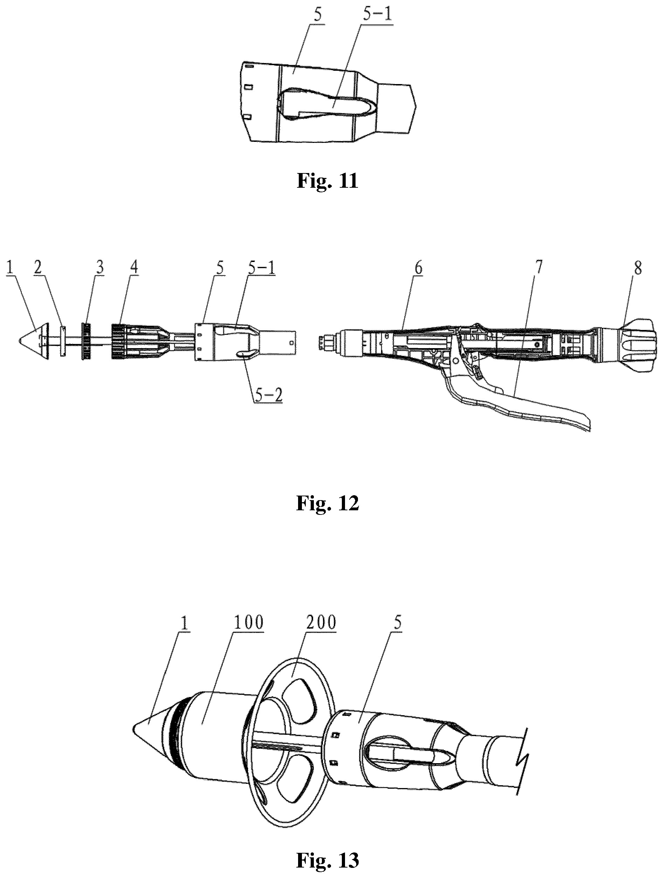

Referring to figures 10 to 12, figure 10 is an outline view of a disposable hemorrhoid stapler according to the present application, figure 11 is a partial enlarged schematic view of a staple cartridge sleeve in figure 10, and figures 12 is an exploded schematic view of an internal structure of the stapler shown in figure 10.

As shown in the figures, the disposable hemorrhoid stapler according to the present application includes an anvil assembly, a staple cartridge assembly and a gun body assembly. The anvil assembly is located in the front of the hemorrhoid stapler and is connected to the gun body assembly via a connecting rod passing through the staple cartridge assembly. The anvil assembly includes a cone-shaped anvil 1. A circular groove is provided in the anvil 1 to receive a cutter gasket ring 2. The staple cartridge assembly includes a staple cartridge 3, a staple-pushing piece 4 located behind the staple cartridge 3, and a staple cartridge sleeve 5. The staple cartridge 3 and the staple-pushing piece 4 are mounted inside the staple cartridge sleeve 5. The staple cartridge sleeve 5 is provided with a thread-hooking hole 5-2 through which a purse string can be pulled out and a purse-string sutured hemorrhoid tissue can be pulled into a cavity of the staple cartridge assembly. Further, the staple cartridge assembly includes an annular cutter (not shown). The annular cutter is mounted in the staple-pushing piece 4, and can move together with the staple-pushing piece 4. The gun body assembly 6 is located at a middle portion of the hemorrhoid stapler. A movable handle 7 is provided below the gun body assembly 6, and an adjusting nut 8 is provided at the tail of the hemorrhoid stapler. The anvil 1 can be driven to be adjacent to or away from the staple cartridge assembly by rotating the adjusting nut 8.

Herein, the staple cartridge 3, the staple-pushing piece 4 and the staple cartridge sleeve 5 are all made of transparent materials, such that it is possible for a surgeon to clearly observe whether there are lost staples in the staple cartridge, and to clearly observe the quantity and uniformity degree of the hemorrhoid tissue entering into the cavity of the staple cartridge assembly during a surgical operation.

A pair of enlarged operating holes 5-1 is provided in an outer sleeve body of the staple cartridge sleeve 5. A surgical clamp may be operated at the operating holes 5-1. It is possible to further observe the quantity of the hemorrhoid tissue entering into the cavity of the hemorrhoid stapler through the operating holes 5-1, and to adjust, by the surgical clamp, the hemorrhoid tissue to be uniform, such that the surgical operation is safer and more reliable, thereby avoiding corresponding complication.

In the following part, a method for operating the anorectal surgical instrument according to the present application will be briefly described with reference to the drawings.

Referring to figure 13, figure 13 is a schematic view of the assembly of the disposable hemorrhoid stapler and the anal dilator according to the present application. The external light source assembly is specially omitted in figure 13 in order to clearly show the fitting relationship between the disposable hemorrhoid stapler and the anal dilator.

In use, firstly, the surgeon uses three non-invasive forceps to perform fixing and outspreading operation at three points of the anus where fewer hemorrhoidal masses are prolapsed and extroversion of the anal membrane is slight. The anal dilator is sleeved on a suturing device which in turn is sleeved on an anorectal introducer, such that the anal dilator and the suturing device can be guided into an anorectum together with the anorectal introducer. After the anorectal introducer is removed, a prolapsed mucosa may fall in a semicircular tubular opening of the suturing device. When an illumination device is switched on by pressing a power switch on the anal dilator, it is possible to observe the dentate line in the anorectum through the transparent cylindrical body of the anal dilator. The suturing device may be rotated such that the opening of the suturing device is aligned with the hemorrhoid tissue, and a circle of purse-string suture is performed at a position more than 3 centimeters to 4 centimeters above the dentate line, and only passes through the mucosa and the submucosa. Then, the circular hemorrhoid stapler is rotated to be open to a maximum position and is guided in through the anal dilator, such that the anvil of the hemorrhoid stapler goes deep to reach the upper end of the purse-string suture, and then the purse-string suture is knotted and tightly tied to a central rod of the stapler. A tail end of the purse-string suture is pulled out through the thread-hooking hole of the hemorrhoid stapler by a thread-hooking device, and the tail end of the suture pulled out of the stapler is knotted or is fixed by forceps. Then, the adjusting nut at the tail end of the stapler is rotated such that the anvil is adjacent to the staple cartridge assembly, and at the same time, the suture is pulled properly such that the prolapsed mucosa layer is entirely placed in the cavity of the staple cartridge assembly of the hemorrhoid stapler. At the same time, the hemorrhoid tissue in the cavity can be adjusted with the surgical clamp to ensure the uniformity of the hemorrhoid tissue in the cavity. After the stapler is completely closed, check whether the hemorrhoid tissue is appropriate or not. Then, the movable handle is pressed to actuate and simultaneously resects the prolapsed mucosa in the cavity, and the stapler exits finally.

In the anorectal surgical instrument according to the present application, the anal dilator is provided with an external light source assembly, and the cylindrical body of the anal dilator is made of transparent materials. In addition, the staple cartridge, the staple-pushing piece and the staple cartridge sleeve of the hemorrhoid stapler are also made of transparent materials. Thus, during a surgical operation, a head portion of the hemorrhoid stapler can be inserted deep inside of the cylindrical body of the anal dilator, and when the power switch on the anal dilator is pressed to switch on the illumination device, rays of the visible light not only can illuminate the entire operative field, but also can transmit through the staple cartridge sleeve to illuminate the staple cartridge and the staple-pushing piece inside the staple cartridge sleeve. Thus, it is possible for the surgeon to clearly observe the status of the staples mounted in the staple cartridge and the situation of the quantity and uniformity of the hemorrhoid tissue entering the cavity of the staple cartridge assembly, so as to assist the surgeon to make determination whether to actuate, which greatly increases the safety and success rate of the surgical operation.

The anorectal surgical instrument and the anal dilator according to the present application have been described above in detail. Specific examples have been employed herein to describe the principle and embodiments of the present application. The above description of the embodiments is only used to help understand the present application. It should be noted that, those skilled in the art may make many improvements and modifications to the present application without departing from the principle of the present application, and these improvements and modifications should also be deemed to fall into protection scope of the present application defined by claims.

## Claims

1. An anal dilator comprising a cylindrical body (100), the cylindrical body (100) being provided on its upper end with an elastic enlarged edge (200),
wherein the anal dilator further comprises an external light source assembly (300) for illuminating an operative field, and the external light source assembly (300) is connected to the enlarged edge (200); **characterised in that** a wall of the cylindrical body (100) is of a double-layer structure being capable of guiding and transmitting light, and a gap inside the double-layer structure forms a light channel (101).

2. The anal dilator according to claim 1, wherein the wall of the cylindrical body (100) is transparent.

3. The anal dilator according to claim 1, wherein a width of the gap at a lower end of the wall of the cylindrical body (100) decreases and light is emitted from the gap at the lower end.

4. The anal dilator according to any one of claims 1 to 3, wherein a wedge-shaped light-concentrating head (301) is provided at a front end of a luminescent light source of the external light source assembly (300), and is insertedly fitted in a wedge-shaped hole (201) in the enlarged edge (200).

5. An anorectal surgical instrument, comprising a disposable hemorrhoid stapler, the anal dilator according to any one of claims 1 to 4 and a thread-hooking device, wherein the anal dilator and the thread-hooking device are used together with the hemorrhoid stapler.

6. The anorectal surgical instrument according to claim 5, wherein the disposable hemorrhoid stapler comprises a cone-shaped anvil assembly located at a front end of the stapler, a staple cartridge assembly located behind the anvil assembly, a gun body assembly (6) located at a middle portion of the stapler, a handle assembly located at a rear portion of the stapler, and an adjusting nut component (8) located at a tail portion of the stapler, and wherein the staple cartridge assembly comprises a staple cartridge (3), a staple-pushing piece and a staple cartridge sleeve (5) which are all made of transparent materials.

7. The anorectal surgical instrument according to claim 6, wherein a pair of enlarged operating holes (5-1) is provided in an outer sleeve body of the staple cartridge sleeve (5).

## Patentansprüche

1. Analer Dilatator, umfassend einen zylindrischen Körper (100), wobei der zylindrische Körper (100) an seinem oberen Ende mit einem elastischen vergrößerten Rand (200) versehen ist,
wobei der anale Dilatator ferner eine externe Lichtquellenanordnung (300) zum Beleuchten eines Operationsfeldes umfasst, und die externe Lichtquellenanordnung (300) mit dem vergrößerten Rand (200) verbunden ist; **dadurch gekennzeichnet, dass** eine Wand des zylindrischen Körpers (100) aus einer Doppelschichtstruktur besteht, die in der Lage ist, Licht zu leiten und zu übertragen, und ein Spalt innerhalb der Doppelschichtstruktur einen Lichtkanal (101) bildet.

2. Analer Dilatator nach Anspruch 1, wobei die Wand des zylindrischen Körpers (100) lichtdurchlässig ist.

3. Analer Dilatator nach Anspruch 1, wobei eine Breite des Spaltes an einem unteren Ende der Wand des zylindrischen Körpers (100) abnimmt und Licht aus dem Spalt an dem unteren Ende ausgestrahlt wird.

4. Analer Dilatator nach einem der Ansprüche 1 bis 3, wobei ein keilförmiger lichtkonzentrierender Kopf (301) an einem vorderen Ende einer lumineszierenden Lichtquelle der externen Lichtquellenanordnung (300) vorgesehen ist und in ein keilförmiges Loch (201) in dem vergrößerten Rand (200) durch Einsetzen eingepasst ist.

5. Anorektales chirurgisches Instrument, umfassend eine wegwerfbare Hämorrhoiden-Klammervorrichtung, den analen Dilatator nach einem der Ansprüche 1 bis 4 und eine Fadeneinhakvorrichtung,
wobei der anale Dilatator und die Fadeneinhakvorrichtung zusammen mit der Hämorrhoiden-Klammervorrichtung verwendet werden.

6. Anorektales chirurgisches Instrument nach Anspruch 5, wobei die wegwerfbare Hämorrhoiden-Klammervorrichtung eine kegelförmige Ambossanordnung, die an einem vorderen Ende der Klammervorrichtung angeordnet ist, eine hinter der Ambossanordnung angeordnete Klammer-Kartuschenanordnung, eine Pistolenkörperanordnung (6), die an einem mittleren Abschnitt der Klammervorrichtung angeordnet ist, eine Griffanordnung, die an einem hinteren Abschnitt der Klammervorrichtung angeordnet ist, und eine Einstellmutterkomponente (8) umfasst, die an einem hinteren Abschnitt der Klammervorrichtung angeordnet ist, und wobei die Klammer-Kartuschenanordnung eine Klammerkartusche (3), ein Klammerschubstück und eine Klammerkartuschenhülse (5) umfasst, die alle aus lichtdurchlässigen Materialien hergestellt sind.

7. Anorektales chirurgisches Instrument nach Anspruch 6, wobei ein Paar vergrößerter Betriebsöffnungen (5-1) in einem äußeren Hülsenkörper der Klammerkartuschenhülse (5) vorgesehen ist.

## Revendications

1. Dilatateur anal comprenant un corps cylindrique (100), le corps cylindrique (100) étant doté sur son extrémité supérieure d'un bord élargi élastique (200),
dans lequel le dilatateur anal comprend en outre un ensemble de source de lumière externe (300) pour éclairer un champ opératoire, et l'ensemble de source de lumière externe (300) est connecté au bord élargi (200) ;
**caractérisé en ce**
**qu'**une paroi du corps cylindrique (100) est d'une structure à double couche étant capable de guider et transmettre la lumière, et un espace vide à l'intérieur de la structure à double couche forme un canal de lumière (101).

2. Dilatateur anal selon la revendication 1, dans lequel la paroi du corps cylindrique (100) est transparente.

3. Dilatateur anal selon la revendication 1, dans lequel une largeur de l'espace vide à une extrémité inférieure de la paroi du corps cylindrique (100) décroît et de la lumière est émise à partir de l'espace vide à l'extrémité inférieure.

4. Dilatateur anal selon l'une quelconque des revendications 1 à 3, dans lequel une tête de concentration de lumière à bord cunéiforme (301) est prévue à une extrémité avant d'une source de lumière luminescente de l'ensemble de source de lumière externe (300), et est logée par insertion dans un orifice cunéiforme (201) dans le bord élargi (200).

5. Instrument chirurgical ano-rectal, comprenant une agrafeuse à hémorroïdes jetable, le dilatateur anal selon l'une quelconque des revendications 1 à 4 et un dispositif à fil à crochetage, dans lequel le dilatateur anal et le dispositif à fil à crochetage sont utilisés conjointement avec l'agrafeuse à hémorroïdes.

6. Instrument chirurgical ano-rectal selon la revendication 5, dans lequel l'agrafeuse à hémorroïdes jetable comprend un ensemble de butée conique situé à une extrémité avant de l'agrafeuse, un ensemble de cartouche d'agrafes situé derrière l'ensemble de butée, un ensemble de corps de pistolet (6) situé à une partie centrale de l'agrafeuse, un ensemble de poignée situé à une partie arrière de l'agrafeuse, et un composant d'écrou d'ajustement (8) situé sur une partie de queue de l'agrafeuse, et dans lequel l'ensemble de cartouche d'agrafes comprend une cartouche d'agrafes (3), une pièce à pousser les agrafes et un manchon de cartouche d'agrafes (5) qui sont tous faits de matériaux transparents.

7. Instrument chirurgical ano-rectal selon la revendication 6, dans lequel une paire d'orifices fonctionnels élargis (5-1) est prévue dans un corps de manchon extérieur du manchon de cartouche d'agrafes (5).
